# EUROPÄISCHE PATENTANMELDUNG

(11) **EP 1 964 567 A1**
(43) Veröffentlichungstag der Anmeldung: **03.09.2008**
(21) Anmeldenummer: 07004183.5
(22) Anmeldetag: 28.02.2007
(51) Int. Cl.: A61K 31/565, A61P 25/06

(54) **Pharmazeutische Zubereitung zur Behandlung der Migräne**

(71) Anmelder: Joos, Catriona, 70563 Stuttgart (DE)
(72) Erfinder: Joos, Catriona, 70563 Stuttgart (DE)
(74) Vertreter: Jacobi, Markus Alexander

(57) **Zusammenfassung**

Die Verwendung von Norethisteron und Norethisteron-Estern zur Herstellung eines Arzneimittels zur Behandlung von Migräne und Krankheiten mit ähnlicher Symptomatik ermöglicht eine schonende Therapie bei niedriger Wirkstoff-Dosierung.

## Beschreibung

Die vorliegende Erfindung betrifft eine pharmazeutische Zubereitung zur Behandlung der Migräne sowie weiterer Erkrankungen, die auf einem Progesteron-Mangel basieren. Hierzu gehören u. a. Migräne mit oder ohne Aura, Spannungskopfschmerzen mit oder ohne Nackenkrämpfe, Schlafstörungen, Depressionen, Panikattacken, übermäßiges schwitzen und verwandte Krankheitsbilder.

Unter Migräne versteht man eine chronisch-rezidivierende, häufig anfallsweise auftretende Krankheit, die in der Regel mit Kopfschmerzen wechselnder Stärke und Andauer einhergeht. Sie tritt häufiger bei Frauen auf als bei Männern und manifestiert sich klinisch in unterschiedlichen Formen, beispielsweise als Migräne ohne Aura mit langsam anwachsendem Kopfschmerz oder als Migräne mit Aura, die mit oftmals neurologischen Ausfallerscheinungen einhergeht, z. B. eingeschränkter kognitiver Wahrnehmung und Lichtscheue.

Während die Ursachen der Migräne weitgehend unbekannt sind, wurden in den letzten Jahren verschiedene Behandlungsverfahren entwickelt, um die typischen Symptome beim Menschen wie Übelkeit, Erbrechen, Kopfschmerz, Gefäßstörungen und andere abzuschwächen. Bei der Behandlung von akuten Migräneattacken kommt bislang beispielsweise eine Kombination eines motilitätssteigernden Antiemetikums zusammen mit einem nicht-opioiden Analgetikum in Frage, was jedoch zu unerwünschten Nebenwirkungen führt.

Bei länger andauernden Migräneattacken haben sich die so genannten Triptane wie beispielsweise Sumatriptan und Rizatriptan als hilfreich erwiesen, ohne jedoch eine länger anhaltende Befreiung von Symptomen bei allen Patientengruppen gewährleisten zu können.

Eine Aufgabe der vorliegenden Erfindung ist es, eine wirksamere und nebenwirkungsfreiere Behandlung von Migräne sowie weiterer Erkrankungen, die auf einem Progesteron-Mangel basieren zu ermöglichen.

Es wurde überraschend gefunden, dass bestimmte Nor-testosteron-Derivate bereits in geringer Dosierung zur wirksamen und andauernden Behandlung und Prophylaxe von Migräne, von Spannungskopfschmerzen (mit oder ohne Nackenkrämpfe) und verwandter Krankheitsbilder eingesetzt werden können, wohingegen andere Gestagene nicht diese gewünschte Aktivität zeigen.

Bereits seit Jahrzehnten ist die Herstellung von Norethisteron bekannt, siehe zum Beispiel US-Patent 2,744,122 oder US-Patent 2,849,462.

Norethisteron (auch 19-Nortesteron bzw. 19-Nor-17α-Ethinyl-17β-hydroxy-pregn-4-en genannt; CAS-Nr. 0000068-22-4) kann über die Alkoholfunktion in Position 17 mit gängigen pharmazeutisch verträglichen Säuren wie z. B. Essig-, Önanth-, Malein-, Fumar-, Zitronen-, Benzolsulfon-, Benzoe-, Ethansulfon-, Glucon-, Glykol-, Milch-, Lactobion-, Malein-, Äpfel-, Methansulfon-, Bernstein-, p-Toluolsulfon- oder Weinsäure verestert werden.

Norethisteron-Acetat wie auch sein Metabolit Norethisteron, werden bislang in erster Linie zur hormonalen Steuerung des monatlichen Zyklus sowie zur Behandlung der Mastopathia cystica bei geschlechtsreifen Frauen eingesetzt. Im Handel sind z. B. Tabletten enthaltend 1 mg oder 5 mg Norethisteron-Acetat sowie Injektionslösungen zur parenteralen Applikation enthaltend 200 mg Norethisteron-Enantat in Rizinusöl erhältlich.

Norethisteron-Acetat ist ein synthetisches Sexualhormon und gehört zur Gruppe der Gestagene (Gelbkörperhormone). Diese regulieren zusammen mit den Östrogenen den Monatszyklus der Frau sowie den Verlauf einer Schwangerschaft. Norethisteron wird seit Jahren zur Behandlung von u. a. Menstruationsbeschwerden, Mastopathie (gutartige Umbauvorgänge des Brustgewebes) oder Endometriose (Wucherungen der Gebärmutterschleimhaut) eingesetzt.

Es wurde nun überraschend gefunden, dass insbesondere Norethisteron-Acetat und Norethisteron-Enantat (der Ester der Onanthsäure) zur nebenwirkungsarmen Behandlung von Migräne, sowie weiterer Erkrankungen, die auf einem Progesteron-Mangel basieren, z. B. Migräne mit oder ohne Aura, Spannungskopfschmerzen mit oder ohne Nackenkrämpfe, Schlafstörungen, Depressionen, Panikattacken, übermäßiges Schwitzen und verwandte Krankheitsbilder eingesetzt werden können.

Norethisteron, seine Ester und Salze können nach in der Literatur bekannten Verfahren hergestellt werden.

Zum Einsatz kommen auch physiologisch verträgliche Salze des Norethisterons und seiner Ester, wobei unter physiologisch verträglichen Salzen sowohl organische als auch anorganische Salze verstanden werden (siehe Remington Pharmaceutical Sciences, 17. Auflage, Seite 1414 von 1985).

Erfindungsgemäß kommen die Wirkstoffe bevorzugt als pharmazeutische Zubereitung zum Einsatz, die Norethisteron und/oder einen Ester des Norethisterons bzw. eines seiner Salze enthält sowie mindestens einen physiologisch geeigneten Hilfsstoff. Diese Komponenten können gemischt werden und anschließend in eine geeignete Form gebracht werden.

Als Säure-Komponente in Norethisteron-Estern kommen physiologisch nicht störende Säuren wie die beispielhaft oben genannten organischen Säuren in Frage, vorzugsweise wird jedoch Norethisteron-Acetat oder Norethisteron-Enantat verwendet.

Die erfindungsgemäße Zubereitung wird im Allgemeinen oral, lokal oder parenteral verabreicht.

Geeignete feste oder flüssige galenische Zubereitungsformen sind beispielsweise Tabletten, Granulate, Pulver, Dragees, Kapseln, Mikrokapseln, Zäpfchen, Sirupe, Emulsionen, Suspensionen, Tropfen oder injizierbare Lösungen in Ampullenform sowie Präparate mit protrahierter Wirkstoff-Freigabe wie Transdermale Therapeutische Systeme (TTS) und Gele zur transkutanen Resorption.

Bei der Herstellung der Zubereitung kommen üblicherweise physiologisch geeignete Hilfsmittel wie Träger-, Binde-, Überzugs-, Quellungs-, Schmiermittel sowie Geschmacksstoffe, Süßungsmittel oder Lösungsvermittler zum Einsatz. Als häufig verwendete Träger und Hilfsstoffe werden beispielsweise Magnesiumcarbonat, Titanoxid, Lactose, Mannit, andere Zucker, Milcheiweiß, Gelatine, Stärke, Zellulose und ihre Derivate, tierische und pflanzliche Öle, Polyethylenglykole, sowie Lösungsmittel wie Wasser, Alkohol, Glycerin genannt.

Vorzugsweise werden die pharmazeutischen Zubereitungen in Dosiereinheiten hergestellt und verabreicht, wobei jede Einheit als aktiven Bestandteil eine bestimmte Dosis von Norethisteron, Norethisteron-Ester und/oder ihrer Salze enthält.

Bei festen Dosiereinheiten wie Kapseln oder Tabletten kann diese Dosis beispielsweise 0,2 mg bis 5 mg, bevorzugt 0,25 mg bis 2,5 mg, insbesondere 0,25 mg bis 1,0 mg betragen. Die Dosierung von 0,5 mg Wirkstoff pro Einheit hat sich besonders bewährt.

Besonders bei lokalen Applikationsformen wie beispielsweise Sprays, Pflaster, Transdermalen Therapeutischen Systemen oder Implantaten kann eine Dosierung von zum Beispiel 0,25 bis 250 mg, vorzugsweise 2,5 bis 20 mg zum Einsatz kommen.

Die zu verabreichende Tagesdosis ist abhängig vom Körpergewicht, Geschlecht, Alter der Person und dem allgemeinen bzw. klinischen Zustand des Patienten.

Die Verabreichung der Tagesdosis kann zwar als Einmalgabe in Form einer einzelnen Dosiereinheit erfolgen, es hat sich jedoch als vorteilhaft erwiesen, kleinere Dosiereinheiten durch Mehrfachgabe zum Einsatz zu bringen.

Besonders vorteilhaft hat sich beim Einsatz von oralen Zubereitungen die Vergabe einer Dosis von 0,25 mg bis 0,75 mg, bevorzugt 0,4 bis 0,6 mg, insbesondere 0,5 mg des Wirkstoffes alle 3 bis 5 Stunden, insbesondere alle 4 Stunden erwiesen. Durch dieses Applikationsschema kann beispielsweise ein beschwerdefreier Nachtschlaf (auch ohne Unterbrechungen der Nachtruhe) erreicht werden.

Die Erfindung betrifft insbesondere die Verwendung von Norethisteron und/oder Norethisteron-Ester bzw. ihren Salzen zur Behandlung bzw. zur Herstellung eines Arzneimittels zur Behandlung von Migräne, von Spannungskopfschmerzen (mit oder ohne Nackenkrämpfe) und verwandter Krankheitsbilder. Die Arzneimittel werden in der Regel dadurch hergestellt, dass man Norethisteron und/oder Norethisteron-Ester bzw. ihren Salzen mit einem oder mehreren physiologisch geeigneten Hilfsstoffen in eine geeignete Darreichungsform bringt.

Auch der Einsatz von Norethisteron und/oder Norethisteron-Ester bzw. ihren Salzen zur Behandlung von Muskelschmerzen in Rücken und/oder Beinen, von plötzlichem starken Schwitzen (z. B. Auftreten von Belastungs-unabhängigen Hitzewallungen), Schlafstörungen (z. B. Einschlaf- oder Durchschlafstörungen, zu früher Aufwachen), übermäßiger Ängstlichkeit (Panik), und Depressionen ist möglich.

Da es von Gestagenen wie Progesteron, Levonorgestrel und Gestoden bekannt war, dass sie ein breites Spektrum von Nebenwirkungen haben, wie beispielsweise die Erzeugung von Übelkeit, Erbrechen und Kopfschmerzen, war es besonders überraschend festzustellen, dass der Testosteronabkömmling Norethisteron und insbesondere dessen Acetat und Enantat zur wirksamen Bekämpfung von Migräne und der Progesteron-Mangelsymptomatik eingesetzt werden können.

Während sich Norethisteron und/oder die Norethisteron-Ester insbesondere bei der Behandlung von Frauen als hilfreich erwiesen hat, kann eine Norethisteron und/oder Norethisteron-Ester enthaltende Zubereitung auch beim Mann eingesetzt werden.

Erkrankungen wie Migräne, Spannungskopfschmerzen mit oder ohne Nackenkrämpfe, Fibromyalgie (Verkrampfung von Muskeln), Parkinson oder auch Restless-Leg-Syndrom lassen sich durch den Einsatz von Norethisteron bzw. -Ester erfolgreich behandeln.

Zur Therapie der oben genannten Zustände können die Verbindungen selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05 % bis 95 Gew.-% des Wirkstoffs enthalten kann. Weitere pharmazeutisch aktive Substanzen können ebenfalls vorhanden sein, einschließlich weiterer Steroide. Die erfindungsgemäßen pharmazeutischen Zusammensetzungen können nach einer der bekannten pharmazeutischen Methoden hergestellt werden, die im Wesentlichen darin bestehen, dass die Bestandteile mit pharmakologisch verträglichen Träger- und/oder Hilfsstoffen gemischt werden.

Erfindungsgemäße pharmazeutische Zusammensetzungen (Zubereitungen) sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphtalat, Hydroxipropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung enthalten; als Pulver oder Granulate; als Lösung der Suspension in einer wässrigen oder nichtwässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im Allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung mit einem Geschmacksstoff enthalten, üblicherweise Saccharose und Gummi arabicum oder Tragant, und Pastillen, die die Verbindung in einer inerten Basis wie Gelatine und Glycerin oder Daccharose und Gummi arabicum umfassen.

Geeignete pharmazeutische Zusammensetzungen für die parenterale Verabreichung umfassen vorzugsweise sterile wässrige Zubereitungen einer Verbindung, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer.

Die oben genannten Verbindungen können auch in Kombination mit weiteren Wirkstoffen verabreicht werden.

Als weitere Wirkstoffe für die Kombinationspräparate sind z.B. geeignet: Migränemittel, die in der "Roten Liste 2005" (Deutsches Arzneimittelverzeichnis) genannt sind. Sie können mit den erfindungsgemäßen Verbindungen insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten erfolgen, worin mehrere Wirkstoffe in einer pharmazeutischen Zubereitung vorliegen.

Die vorliegende Erfindung betrifft auch die Herstellung einer pharmazeutischen Zubereitung enthaltend Norethisteron bzw. dessen Ester, insbesondere Norethisteronacetat und -enantat zur Behandlung von Migräne, sowie weiterer Erkrankungen, die auf einem Progesteron-Mangel basieren. Hierzu gehören u. a. Migräne mit oder ohne Aura, Spannungskopfschmerzen mit oder ohne Nackenkrämpfe, Schlafstörungen, Depressionen, Panikattacken, übermäßiges Schwitzen. Ferner sind die Zubereitungen auch einsetzbar zu Behandlung von Fibromyalgie, Parkinson und/oder Restless-Leg-Syndrom.

Von der Parkinson-Erkrankung wird bislang angenommen, dass sie durch eine Degeneration der dopaminergen Neuronen verursacht wird. Dies führt zu einem Ungleichgewicht der Neurotransmitter, insbesondere Acetylcholin und Dopamin. Die Parkinsonerkrankung äußert sich u. a. durch Symptome wie Tremor, Bradykinesie, Störungen beim Gang und Sprachstörungen.

In der Regel tritt die Erkrankung bei Männern und Frauen gleichermaßen häufig im mittleren Alter auf. Da die bisherigen Therapieansätze mit Levo-Dopa zu schweren Nebenwirkungen und einer immer höheren Dosierung führen können, besteht ein großer Bedarf an neuartigen Behandlungsmethoden mit geringeren Nebenwirkungen.

Es wurde nun überraschend gefunden, dass sich Norethisteron-Acetat bzw. -Enantat zur wirksamen Therapie und Prophylaxe von Parkinson eignet.

Auch eine Behandlung des Restless-Leg-Syndroms (RLS) durch Anwendung einer Zubereitung enthaltend Norethisteron-Acetat oder Norethisteron-Enantat ist möglich. Bei RLS handelt es sich um eine neurologische Erkrankung, die insbesondere mit einer Missempfindung in den Beinen und einem starken Bewegungsdrang einhergeht. Diese Symptome können ähnlich wie eine Migräne zu einer starken Beeinträchtigung des Nachtschlafes führen. Durch Anwendung von Norethisteron-Acetat, beispielsweise mehrfach täglich in niedriger Dosierung angewandt, lassen sich die Symptome von RLS wirksam behandeln.

Die vorliegende Erfindung betrifft ferner auch eine Zubereitung für die transdermale Anwendung, insbesondere ein Transdermales Therapeutisches System (TTS) zur Verabreichung von Norethisteron bzw. dessen Ester durch die Haut über einen längeren Zeitraum. Ein derartiges TTS besteht in der Regel aus einer Fixierungshilfe und einer Matrix, welche den Wirkstoff enthält.

Das TTS bietet den Vorteil einer kontinuierlichen Arzneistoffzufuhr unter Umgehung des Magen-Darmtraktes. Aus Kostengründen und für eine bessere Patientencompliance bietet sich eine Bereitstellung eines Norethisteron-Pflasters zur mehrtägigen Verwendung an. Da es bei der Behandlung von Migräne bzw. des Spannungskopfschmerz mit oder ohne Nackenkrämpfe und anderer Erkrankungen, bedingt durch Progesteronmangel, mittels Norethisteron/-Acetat auf eine möglichst konstante Zuführung geringer Mengen von Norethisteron bzw. Norethisteron-Acetat ankommt, empfiehlt sich die Verwendung eines modernen Transdermalen Therapeutischen Systems, beispielsweise eines TTS (z. B. bestehend aus einer Methacrylat-polymer-Matrix, die den Wirkstoff enthält, ein oder mehreren Abdeckschichten sowie einer haltbaren Fixierung). Dabei wird bevorzugt ein TTS eingesetzt, das über ein oder mehrere Tage hinweg eine Dosierung von 0,5 bis 8 mg des Wirkstoffes, insbesondere 1,5 bis 3 mg pro Tag, abgibt, wobei diese Freisetzung insbesondere mit nahezu konstanter Abgaberate erfolgt.

Die vorliegenden Beispiele sollen die Erfindung näher erläutern, ohne dabei die Erfindung in irgendeiner Weise zu begrenzen.

### Beispiel 1

Es wird eine Tablette hergestellt, die neben 1 mg Norethisteron-Acetat die gängigen Zusatz- und Hilfsstoffe wie Lactose, Magnesiumstearat, Talkum, Caboxymethylcellulose Natrium Typ H, Gelatine und Kartoffelstärke enthält.

Eine 37-jährigen Patientin, die seit mehreren Wochen über andauernde Kopfschmerzen sowie Berührungs-Schmerzen der linken Brust klagt, erhält Norethisteron-Acetat als Tablette in der Dosierung 1 mg pro Tag zyklisch, d. h. vom 12. Zyklustag bis zum 22. Zyklustag. Die Patientin ist bereits nach 2 Einnahmetagen von je 1 mg Norethisteron-Acetat als Tablette völlig beschwerdefrei.

Nach Absetzen von Norethisteron-Acetat (einmal täglich, je 1 mg als Tablette) nach dem 22. Zyklustag beginnen die Beschwerden von neuem und verschwinden erst wieder mit der nächsten Einnahme von 1 mg Norethisteron-Acetat am 12. Tag des nächsten Monatszyklus.

Erst nach Einsatz von 1 mg Norethisteron-Acetat täglich ohne Unterbrechungen ist die Patientin vollständig beschwerdefrei. Auch nach längerer Einnahmedauer von täglich 1 mg Norethisteron-Acetat zeigen sich keine Hinweise auf Nebenwirkungen.

Während der Therapie der Migräne und des Spannungskopfschmerzes wird festgestellt, dass auch Muskelschmerzen in Rücken und Beinen sowie Schlaflosigkeit mit anschließenden Depressionen völlig verschwinden. Die geschilderten Probleme gehen häufig einher mit einem Abfall des natürlich vorkommenden Progesteron-Spiegels im Organismus. Die gezeigte Wirksamkeit von Norethisteron-Acetat bei Schlaflosigkeit und Depressionen deutet auf die regulierende Fähigkeit im Bereich des zentralen Nervensystems hin.

Beim Mann findet die Produktion von Progesteron und Estrogenen in den Testes statt. Da Norethisteron androgene Wirksamkeit besitzt, ist der Einsatz auch beim Mann durchaus möglich. Einsatzgebiet ist auch hier beispielsweise die Migräne und Spannungskopfschmerz mit oder ohne Nackenkrämpfe.

### Beispiel 2:

Es wird eine Tablette hergestellt, die neben 0,5 mg Norethisteron-Acetat die gängigen Zusatz- und Hilfsstoffe, wie z. B. Lactose, Magnesiumstearat, Talkum, Carboxymethylcellulose Natrium Typ H, Gelatine und Kartoffelstärke enthält.

Bei einer 50-jährigen Patientin mit seit über 10 Jahren bestehenden Spannungskopfschmerzen mit Nackenkrämpfen, Muskelschmerzen in Rücken und Beinen, Schlaflosigkeit mit nachfolgenden Depressionen, Herzrasen und Panik-Attacken werden verschiedene handelsübliche Gestagen-Monopräparat gestestet, ohne dass eine Linderung der Symptome erreicht werden kann.

Mit Norethisteron-Acetat in einer Dosierung von 0,5 mg alle 4 Stunden oral eingenommen, ist es möglich, eine dauerhafte Beschwerdefreiheit der Patientin zu erreichen.

Die beschriebene Dosierung zeigt dabei keine klinisch relevanten Nebenwirkungen. Auch treten Virilisierungs-Erscheinungen selbst bei längerer Behandlung nicht auf. Der Einsatz von 1 mg/Tag bzw. 4 x 0,5 mg alle 4 Stunden erweist sich im Vergleich zu anderen Gestagen-Monopräparaten als sehr wirkungsvoll bei geringem Nebenwirkungsprofil.

Insbesondere bei der Dosierung von 4 x 0,5 mg Norethisteron-Acetat/Tag sind Wassereinlagerungen im Gewebe minimal. Eine Virilisierung kann nicht beobachtet werden. Veränderte Laborparameter werden auch nach einem längeren Behandlungszeitraum nicht beobachtet.

### Beispiel 3:

Bei einer 40-jährigen Patientin, die wiederholt für mehrere Wochen unter andauernden Kopfschmerzen sowie anderen Migräne-Symptomen leidet, wird Norethisteron-Enantat eingesetzt.

Zur Anwendung kommt Norethisteron-Enantat 200 mg (entsprechend 145.353 mg Norethisteron) als handelsübliche Fertigspritze (Dreimonatsspritze). Die Patientin stellt nach kurzer Zeit fest, dass ihre seit Jahren bestehende Migräne völlig verschwunden ist. Die Wirkung hält mehrere Wochen an.

Der Einsatz von Norethisteron-Acetat oder Norethisteron-Enantat als Depot ist sinnvoll im Sinne einer langfristigen schonenden Hormonersatztherapie. Es treten keine Migräne-Symptome mehr auf und keine Kopfschmerzen. Die Monatsblutung tritt ebenfalls nicht mehr ein.

Der Einsatz von Norethisteron-Estern über Retard-Formulierungen bietet sich an, beispielsweise über ein TTS, das den Wirkstoff in einer langsam-freisetzenden Polymermatrix enthält. Es lässt sich so eine gezielte Freigabe von z. B. 0,5 mg bis 8 mg pro Tag bei guter Patienten-Compliance erreichen.

## Patentansprüche

1. Verwendung von Norethisteron, Norethisteron-Estern und/oder ihren physiologisch verträglichen Salzen zur Herstellung eines Arzneimittels zur Behandlung von Migräne, Spannungskopfschmerzen mit oder ohne Nackenkrämpfen, Schlafstörungen, Depressionen, Panikattacken, übermäßigem Schwitzen beruhend auf einem Progesteron-Mangel, Fibromyalgie, Parkinson und/oder Restless-Leg-Syndrom.

2. Verwendung von Norethisteron, Norethisteron-Estern und/oder ihren physiologisch verträglichen Salzen gemäß Anspruch 1, **dadurch gekennzeichnet, dass** der Wirkstoff als Arzneimittel für eine orale Zubereitung formuliert ist, die 0,25 mg bis 2,5 mg des Wirkstoffs enthält.

3. Verwendung von Norethisteron, Norethisteron-Estern und/oder ihren physiologisch verträglichen Salzen gemäß Anspruch 1 oder 2, **dadurch gekennzeichnet, dass** der Wirkstoff als lokal oder parenteral verabreichbare Zubereitung formuliert ist, die 0,25 mg bis 200 mg des Wirkstoffs enthält.

4. Verwendung von Norethisteron, Norethisteron-Estern und/oder ihren physiologisch verträglichen Salzen gemäß einem der Ansprüche 1 bis 3, **dadurch gekennzeichnet, dass** ein Transdermales Therapeutisches System enthaltend 2,5 mg bis 20 mg des Wirkstoffs verwendet wird.

5. Verwendung von Norethisteron, Norethisteron-Estern und/oder ihren physiologisch verträglichen Salzen gemäß einem der Ansprüche 1 bis 4, **dadurch gekennzeichnet, dass** als Wirkstoff Norethisteron-Acetat und/oder Norethisteron-Enantat eingesetzt wird.

6. Pharmazeutische Zubereitung zur Behandlung von Migräne, Spannungskopfschmerzen mit oder ohne Nackenkrämpfen, Schlafstörungen, Depressionen, Panikattacken, übermäßiges Schwitzen beruhend auf einem Progesteronmangel, Fibromyalgie, Parkinson und/oder Restless-Leg-Syndrom enthaltend 0,25 mg bis 200 mg eines Norethisteron-Esters oder eines physiologisch verträglichen Salzes.

7. Pharmazeutische Zubereitung gemäß Anspruch 6, **dadurch gekennzeichnet, dass** sie 0,25 bis 0,75 mg Norethisteron-Acetat und/oder Norethisteron-Enantat enthält.

8. Pharmazeutische Zubereitung gemäß einem der Ansprüche 6 oder 7, **dadurch gekennzeichnet, dass** sie zur Behandlung von Migräne und/oder Spannungskopfschmerzen mit oder ohne Nackenkrämpfen eingesetzt wird und die Zubereitung 0,4 mg bis 0,6 mg eines Norethisteron-Acetat enthält.

9. Pharmazeutische Zubereitung gemäß einem der Ansprüche 6 bis 8, **dadurch gekennzeichnet, dass** es sich um eine orale Zubereitung handelt.

10. Pharmazeutische Zubereitung gemäß einem der Ansprüche 6 bis 8, **dadurch gekennzeichnet, dass** es sich um eine Zubereitung für eine transdermale Anwendung handelt.

11. Pharmazeutische Zubereitung gemäß einem der Ansprüche 6 bis 9, **dadurch gekennzeichnet, dass** es sich bei der Zubereitung um ein Transdermales Therapeutisches System handelt.

12. Verfahren zur Herstellung einer pharmazeutische Zubereitung gemäß einem der Ansprüche 6 bis 11, **dadurch gekennzeichnet, dass** Norethisteron und/oder ein Ester des Norethisterons bzw. eines seiner Salze sowie mindestens ein physiologisch geeigneter Hilfsstoff gemischt und anschließend in eine geeignete Form gebracht werden.
